(19) Europäisches Patentamt
European Patent Office
Office européen des brevets

(11) **EP 2 397 841 A1**

(12) **EUROPEAN PATENT APPLICATION**
published in accordance with Art. 153(4) EPC

(43) Date of publication:
**21.12.2011 Bulletin 2011/51**

(51) Int Cl.:
***G01N 21/64*** *(2006.01)*

(21) Application number: **10741032.6**

(86) International application number:
**PCT/JP2010/000390**

(22) Date of filing: **25.01.2010**

(87) International publication number:
**WO 2010/092752 (19.08.2010 Gazette 2010/33)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.02.2009 JP 2009028325**

(71) Applicant: **Mitsui Engineering & Shipbuilding Co., Ltd.**
**Chuo-ku**
**Tokyo 104-8439 (JP)**

(72) Inventor: **HAYASHI, Hironori**
**Tamano-shi**
**Okayama 706-8651 (JP)**

(74) Representative: **Hards, Andrew**
**Hards & Franke**
**Patentanwälte Partnerschaft**
**Widenmayerstraße 25**
**80538 München (DE)**

(54) **FLUORESCENCE DETECTION DEVICE AND FLUORESCENCE DETECTION METHOD**

(57) Disclosed herein is a fluorescence detecting device intended to improve the measurement accuracy of a fluorescence relaxation time. The fluorescence detecting device includes a laser light source unit that irradiates a measurement object with laser light, a light-receiving unit that outputs a fluorescent signal of fluorescence emitted by the measurement object irradiated with the laser light, a light source control unit that generates a modulation signal for time-modulating an intensity of the laser light emitted from the laser light source unit by at least two frequency components, and a processing unit that determines a fluorescence relaxation time of the fluorescence emitted by the measurement object by using the fluorescent signal outputted by the light-receiving unit and the modulation signal, wherein the processing unit determines phase delays of the fluorescent signal with respect to the modulation signal at the at least two frequency components, and determines a fluorescence relaxation time at each of the frequency components by using the phase delay, and determines an average fluorescence relaxation time by weighted averaging of the fluorescence relaxation times.

FIG.4

EP 2 397 841 A1

**Description**

TECHNICAL FIELD

The present invention relates to a device for detecting fluorescence by receiving fluorescence emitted by a measurement object irradiated with laser light and processing a fluorescent signal obtained at this time. The present invention also relates to a method for detecting fluorescence by receiving fluorescence emitted by a measurement object irradiated with laser light and processing a fluorescent signal obtained at this time. Particularly, the present invention relates to a fluorescence detecting device to be used in an analyzing device, such as a flow cytometer for use in medical and biological fields, which analyzes a measurement object, such as cells, DNA, or RNA, by identifying the measurement object based on fluorescence emitted by a fluorochrome.

BACKGROUND ART

A flow cytometer used in medical and biological fields includes a fluorescence detecting device that identifies the type of a measurement object by receiving fluorescence emitted by a fluorochrome in the measurement object irradiated with laser light.
More specifically, in the flow cytometer, a suspension liquid containing a measurement object such as a biological material (e.g., cells, DNA, RNA, enzymes, or proteins), labeled with a fluorescent reagent is allowed to flow through a tube together with a sheath liquid flowing under pressure at a speed of about 10 m/sec or less to form a laminar sheath flow. The flow cytometer receives fluorescence emitted by a fluorochrome attached to the measurement object by irradiating the measurement object in the laminar sheath flow with laser light and identifies the measurement obj ect by using the fluorescence as a label.
The flow cytometer can measure, for example, the relative amounts of DNA, RNA, enzymes, proteins, etc. contained in a cell and can quickly analyze their functions. Further, a cell sorter or the like is used to identify a predetermined type of cell or chromosome based on fluorescence and selectively and quickly collect only the identified cells or chromosomes alive.
For example, when a biological material such as DNA is analyzed by a flow cytometer, a fluorochrome is previously attached to the biological material with a fluorescent reagent. The biological material is labeled with the fluorochrome different from a fluorochrome attached to a microbead (which will be described later), and is mixed with a liquid containing microbeads having a diameter of 5 to 20 $\mu$m. Each of the microbeads has a specific structure, such as a carboxyl group, provided on the surface thereof. The specific structure, such as a carboxyl group, acts on and is coupled to a biological material having a certain known structure. Therefore, simultaneous detection of fluorescence derived from the microbead and fluorescence derived from the biological material indicates that the biological material has been coupled to the specific structure of the microbead. This makes it possible to analyze the characteristics of the biological material. In order to prepare various microbeads having different structures for coupling to quickly analyze the characteristics of the biological material, a very wide variety of fluorochromes are required.
Patent Document 1 discloses a fluorescence detecting device that determines the fluorescence relaxation time of fluorescence emitted by a measurement object, such as a microbead, by irradiating the measurement object with laser light whose intensity is modulated at a predetermined frequency. The fluorescence relaxation time varies depending on the type of fluorochrome used, and therefore the type of fluorescence can be identified based on the fluorescence relaxation time, which makes it possible to identify the type of the measurement obj ect.

PRIOR ART DOCUMENT

Patent Document

Patent Document 1: Japanese Patent Application Laid-Open No. 2006-226698

DISCLOSURE OF THE INVENTION

PROBLEMS TO BE SOLVED BY THE INVENTION

The fluorescence detecting device disclosed in Patent Document 1 can quickly and efficiently identify fluorescence based on a fluorescence relaxation time, but the accuracy of fluorescence relaxation time measurement is not always high. For example, when a measurement object includes a microbead or the like that emits fluorescence having a relatively long fluorescence relaxation time exceeding 20 nsec, the accuracy of fluorescence relaxation time measurement is lowered.

It is therefore an object of the present invention to provide a fluorescence detecting device and a fluorescence detecting method which make it possible to achieve higher accuracy of fluorescence relaxation time measurement.

MEANS FOR SOLVING THE PROBLEMS

The inventor of the present invention found that a range of fluorescence relaxation time of a measurement obj ect with higher accuracy varies depending on the modulation frequency of the laser light. In other words, when the fluorescence relaxation time of a measurement object extends over a wide range, the measurement accuracy is to be improved by modulating a intensity of the laser light with multiple frequency components, not with single frequency component.

A fluorescence detecting device of the present invention for receiving fluorescence emitted by a measurement object by irradiating the measurement object with laser light and processing a fluorescent signal obtained at this time, the device comprising: a laser light source unit that irradiates the measurement object with laser light; a light-receiving unit that outputs a fluorescent signal of fluorescence emitted by the measurement object irradiated with the laser light; a light source control unit that generates a modulation signal for time-modulating an intensity of the laser light emitted from the laser light source unit by at least two frequency components; and a processing unit that determines a fluorescence relaxation time of the fluorescence emitted by the measurement object by using the fluorescent signal outputted by the light-receiving unit and the modulation signal, wherein the processing unit determines phase delays of the fluorescent signal with respect to the modulation signal at the two frequency components, and determines a fluorescence relaxation time at each of the frequency components by using the phase delay, and determines an average fluorescence relaxation time by weighted averaging of the fluorescence relaxation times.

A fluorescence detecting method of the present invention by receiving fluorescence emitted by a measurement object by irradiating the measurement object with laser light and processing a fluorescent signal obtained at this time, the method comprising the steps of: irradiating the measurement object with laser light; outputting a fluorescent signal of fluorescence emitted by the measurement object irradiated with the laser light; generating a modulation signal for time-modulating an intensity of the laser light by at least two frequency components; and determining a fluorescence relaxation time of the fluorescence emitted by the measurement obj ect by using the fluorescent signal and the modulation signal, wherein the step of determining a fluorescence relaxation time includes the step of determining phase delays of the fluorescent signal with respect to the modulation signal at the two frequency components, the step of determining a fluorescence relaxation times at each of the frequency components by using the phase delay, and the step of determining an average fluorescence relaxation time by weighted averaging of the fluorescence relaxation times.

EFFECTS OF THE INVENTION

According to the fluorescence detecting device and the fluorescence detecting method of the present invention, it is possible to achieve higher accuracy of fluorescence relaxation time measurement.

BRIEF DESCRIPTION OF THE DRAWINGS

Fig. 1 is a schematic diagram illustrating the structure of one example of a flow cytometer that uses a fluorescence detecting device using intensity-modulated laser light.
Fig. 2 is a schematic diagram illustrating the structure of one example of a laser light source unit for use in the fluorescence detecting device using intensity-modulated laser light.
Fig. 3 is a schematic diagram illustrating the structure of one example of a light-receiving unit for use in the fluorescence detecting device using intensity-modulated laser light.
Fig. 4 is a schematic diagram illustrating the structure of one example of a control/processing unit for use in the fluorescence detecting device using intensity-modulated laser light.
Fig. 5 is a schematic diagram illustrating the structure of one example of an analyzing device for use in the fluorescence detecting device using intensity-modulated laser light.
Fig. 6 is a graph illustrating the amount of change of a phase delay with respect to a fluorescence relaxation time.

EMBODIMENTS FOR CARRYING OUT THE INVENTION

Hereinbelow, a flow cytometer that appropriately uses a fluorescence detecting device according to the present invention using intensity-modulated laser light will be described based on the following embodiments.

First Embodiment

(Overall Structure of Flow Cytometer)

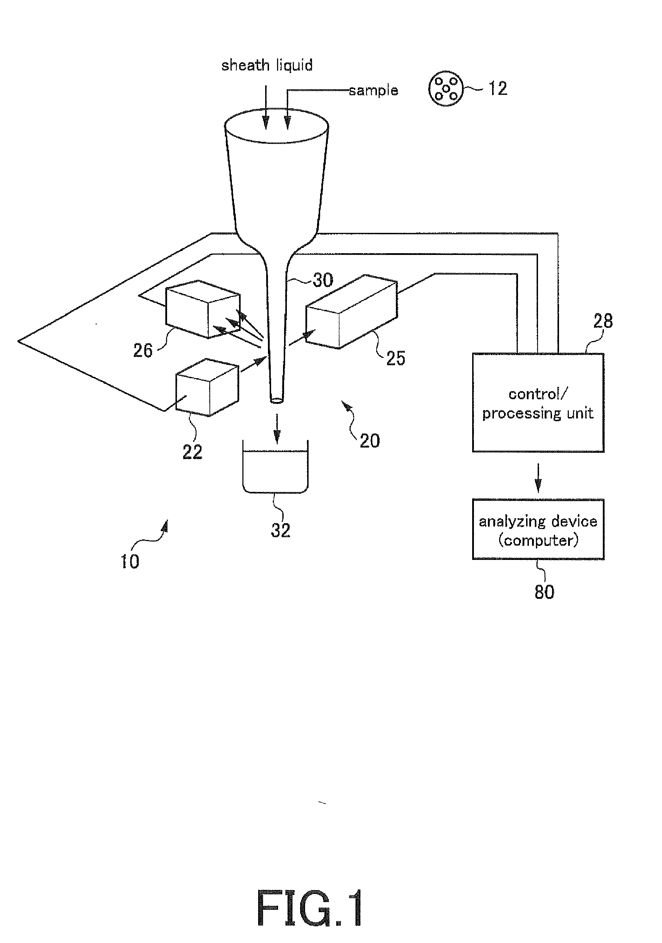

First, the overall structure of a flow cytometer according to a first embodiment will be described with reference to Fig. 1. Fig. 1 is a schematic diagram illustrating the structure of one example of a flow cytometer 10 that uses the fluorescence detecting device according to the present invention using intensity-modulated laser light.

The flow cytometer 10 includes a signal processing unit 20 and an analyzing device (computer) 80. The signal processing device 20 detects and processes a fluorescent signal of fluorescence emitted by a fluorochrome introduced into a sample 12, which is a measurement object such as a microbead or a cell, by irradiation with laser light. The analyzing device (computer) 80 analyzes the measurement object in the sample 12 from processed results obtained by the signal processing device 20.

As will be described later, the signal processing device 20 includes a laser light source unit 22, light-receiving units 25 and 26, a control/processing unit 28, and a tube 30.
The control/processing unit 28 includes a signal generation unit 40, a signal processing unit 42, and a controller 44. The signal generation unit 40 modulates the intensity of laser light emitted from the laser light source unit 22. The signal processing unit 42 identifies a fluorescent signal from the sample 12. The controller 44 manages all the operations of the flow cytometer 10.
The tube 30 allows a sheath liquid forming a high speed flow to flow therethrough together with the samples 12 to form a laminar sheath flow. The laminar sheath flow has a diameter of, for example, 100 $\mu$m and a flow rate of 1 to 10 m/sec. When a microbead is used as the sample 12, the diameter of the microbead is several micrometers to 30 $\mu$m. A collection vessel 32 is provided at the outlet of the tube 30.
The flow cytometer 10 may include a cell sorter to quickly separate a biological material such as specific cells in the sample 12 after irradiation with laser light into different collection vessels.
The laser light source unit 22 emits laser light whose intensity is modulated at a predetermined frequency. The laser light source unit 22 has a lens system so that the laser light is focused on a predetermined position in the tube 30. The sample 12 is measured at a position (measurement point) on which the laser light is focused.

(Laser Light Source Unit)

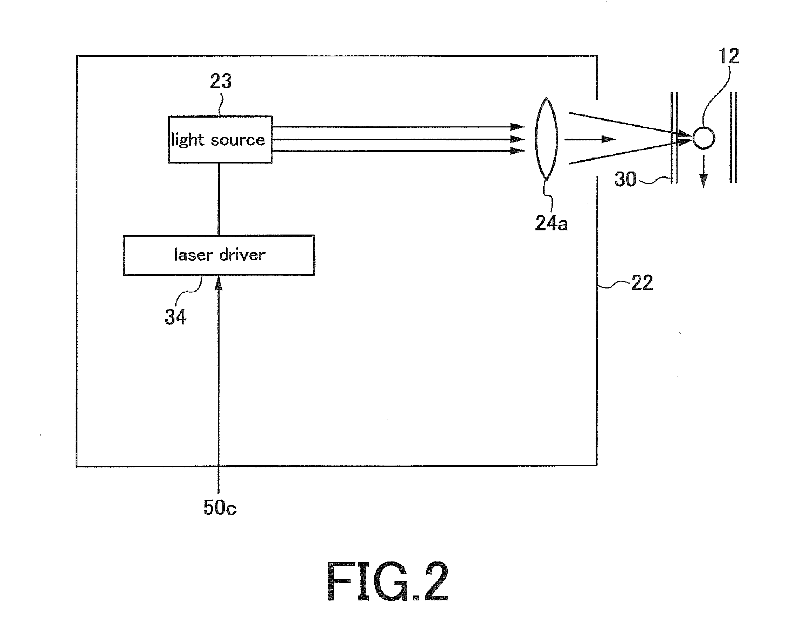

The laser light source unit 22 will be described with reference to Fig. 2. Fig. 2 is a schematic diagram illustrating one example of the structure of the laser light source unit 22.

The laser light source unit 22 includes a light source 23, a lens system 24a, and a laser driver 34. The light source 23 emits CW (continuous-wave) laser light of constant intensity while modulating the intensity of the CW laser light. The lens system 24a focuses laser light emitted from the light source 23 on the measurement point in the tube 30. The laser driver 34 drives the light source 23.

The light source 23 that emits laser light is, for example, a semiconductor laser. The output of the laser light is, for example, about 5 to 100 mW. The laser light has a wavelength within, for example, a visible light band of 350 nm to 800 nm.

The laser driver 34 is connected to the control/processing unit 28. The laser driver 34 generates a driving signal for modulating the intensity of laser light by using a modulation signal including at least two frequencies, and supplies the driving signal to the light source 23.

The fluorochrome to be excited by laser light is attached to the sample 12 (measurement object) such as a biological material or a microbead. When passing through the tube 30 in several microseconds to several tens of microseconds, the sample 12 is irradiated with laser light at the measurement point and emits fluorescence. At this time, the laser light is emitted while being intensity-modulated at two frequencies.

(Light-Receiving Unit)

Referring to Fig. 1 again, the light-receiving unit 25 is arranged so as to be opposed to the laser light source unit 22 with the tube 30 being provided therebetween. The light-receiving unit 25 is equipped with a photoelectric converter that detects forward scattering of laser light caused by the sample 12 passing through the measurement point and outputs a detection signal indicating the passage of the sample 12 through the measurement point. The signal outputted from the light-receiving unit 25 is supplied to the control/processing unit 28, and is used in the control/processing unit 28 as a trigger signal to announce the timing of passage of the sample 12 through the measurement point in the tube 30.

On the other hand, the light-receiving unit 26 is arranged in a direction perpendicular to a direction in which laser light emitted from the laser light source unit 22 travels and to a direction in which the sample 12 moves in the tube 30. The light-receiving unit 26 is equipped with a photoelectric converter that receives fluorescence emitted by the sample 12 irradiated with laser light at the measurement point.

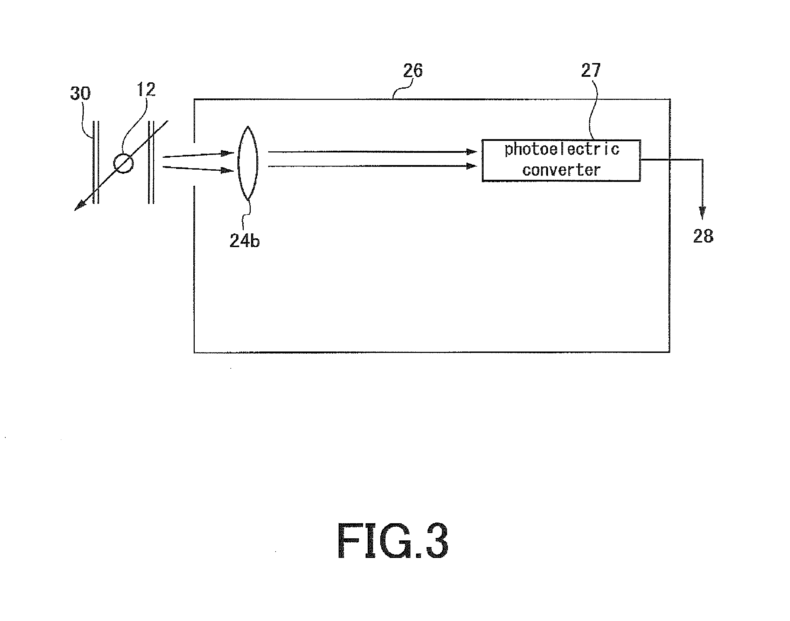

The general structure of the light-receiving unit 26 will be described with reference to Fig. 3. Fig. 3 is a schematic diagram illustrating the structure of one example of the light-receiving unit 26. As illustrated in Fig. 3, the light-receiving unit 26 includes a lens system 24b and a photoelectric converter 27. The lens system 24b focuses a fluorescent signal from the sample 12. The lens system 24b is configured to focus fluorescence received by the light-receiving unit 26 on the light-receiving surface of the photoelectric converter 27.

The photoelectric converter 27 is equipped with, for example, a photomultiplier to convert light received by its photoelectric surface to an electrical signal. The electrical signal (fluorescent signal) converted by the photoelectric converter 27 is supplied to the control/processing unit 28.

(Control/Processing Unit)

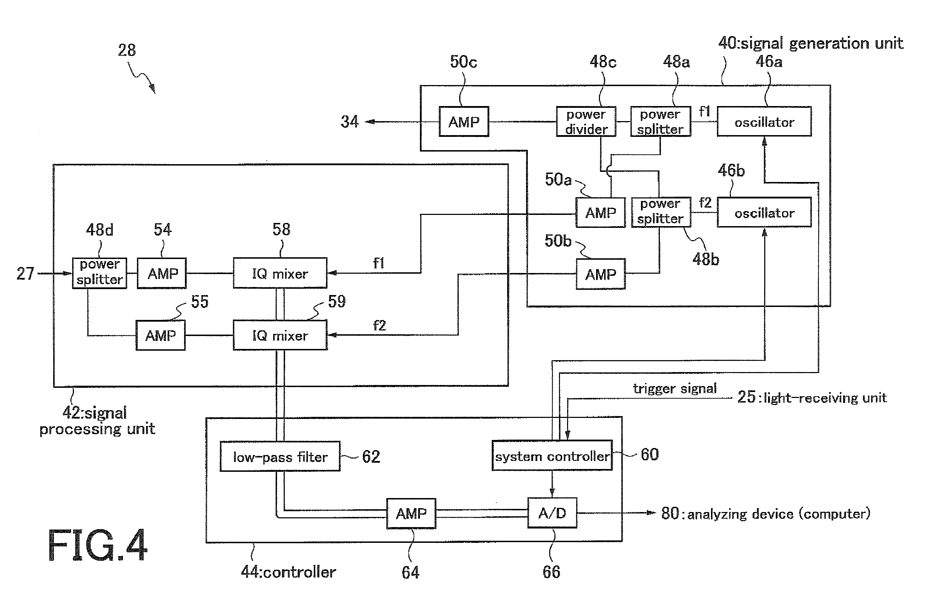

The general structure of the control/processing unit 28 will be described with reference to Fig. 4. Fig. 4 is a schematic diagram illustrating the structure of one example of the control/processing unit 28. The control/processing unit 28 includes the signal generation unit 40, the signal processing unit 42, and the controller 44. The signal generation unit 40 and the controller 44 constitute a light source control unit that generates a modulation signal for modulating the intensity of laser light.

The signal generation unit 40 includes oscillators 46a and 46b, power splitters 48a and 48b, a power divider 48c, and amplifiers 50a, 50b, and 50c. The signal generation unit 40 generates a modulation signal and supplies the modulation signal to the laser driver 34 of the laser light source unit 22 and the signal processing unit 42. As will be described later, the modulation signal supplied to the signal processing unit 42 is used as a reference signal for detecting a fluorescent signal outputted from the photoelectric converter 27.

Each of the oscillators 46a and 46b outputs a sinusoidal signal, but a sinusoidal signal outputted from the oscillator 46a and a sinusoidal signal outputted from the oscillator 46b are different in frequency. The frequency of each of the sinusoidal signals is set to a value in the range of, for example, 1 to 50 MHz. The sinusoidal signal outputted from the oscillator 46a has a frequency of $f_1$ (angular frequency of $\omega_1$), and is split by the power splitter 48a and sent to the power divider 48c and the amplifier 50a. The sinusoidal signal outputted from the oscillator 46b has a frequency of $f_2$ (angular frequency of $\omega_2$), and is split by the power splitter 48b and sent to the power divider 48c and the amplifier 50b. The sinusoidal signal sent from the power splitter 48a to the power divider 48c is combined with the sinusoidal signal sent from the power splitter 48b to the power divider 48c by the power divider 48c to generate a modulation signal. The modulation signal generated by the power divider 48c is amplified by the amplifier 50c and supplied to the laser driver 34.

The signal processing unit 42 uses the fluorescent signal outputted from the photoelectric converter 27 to extract information about the phase delay of fluorescence emitted by the measurement object such as a microbead by irradiation with laser light. The signal processing unit 42 includes a power splitter 48d, amplifiers 54 and 55, and IQ mixers 58 and 59.

The power splitter 48d splits the fluorescent signal outputted from the photoelectric converter 27 and sends the split fluorescent signals to the amplifiers 54 and 55. The amplifier 54 amplifies the split fluorescent signal sent from the power splitter 48d and supplies the amplified fluorescent signal to the IQ mixer 58. The amplifier 55 amplifies the split fluorescent signal sent from the power splitter 48d and supplies the amplified fluorescent signal to the IQ mixer 59. To the IQ mixer 58, the sinusoidal signal having a frequency of $f_1$ and supplied from the amplifier 50a is also supplied as a reference signal. To the IQ mixer 59, the sinusoidal signal having a frequency of $f_2$ and supplied from the amplifier 50b is also supplied as a reference signal.

The IQ mixer 58 is a device that combines the fluorescent signal supplied from the photoelectric converter 27 with the sinusoidal signal having a frequency of $f_1$ and supplied from the signal generation unit 40 as a reference signal. The IQ mixer 59 is a device that combines the fluorescent signal supplied from the photoelectric converter 27 with the sinusoidal signal having a frequency of $f_2$ and supplied from the signal generation unit 40 as a reference signal. More specifically, each of the IQ mixers 58 and 59 multiplies the fluorescent signal (RF signal) by the reference signal to calculate a processing signal including a cos component and a high-frequency component of the fluorescent signal. Further, each of the IQ mixers 58 and 59 multiplies the florescent signal by a signal obtained by shifting the phase of the reference signal by 90° to calculate a processing signal including a sin component and a high-frequency component of the fluorescent signal. The processing signal including the cos component and the processing signal including the sin component are supplied to the controller 44.

The controller 44 includes a system controller 60, a low-pass filter 62, an amplifier 64, and an A/D converter 66.

The system controller 60 gives instructions for controlling the operations of the individual units and manages all the operations of the flow cytometer 10. Further, the system controller 60 controls the oscillators 46a and 46b of the signal generation unit 40 to generate sinusoidal signals having predetermined frequencies.

The low-pass filter 62 removes the high-frequency component from the processing signal which is calculated by the signal processing unit 42 and in which the high-frequency component is added to the cos component, and removes the high-frequency component from the processing signal which is calculated by the signal processing unit 42 and in which the high-frequency component is added to the sin component. As a result, the processing signal of the cos component with a frequency of $f_1$, the processing signal of the cos component with a frequency of $f_2$, the processing signal of the sin component with a frequency of $f_1$, and the processing signal of the sin component with a frequency of $f_2$ are obtained. The amplifier 64 amplifies the processing signals of the cos component and the processing signals of the sin component. The A/D converter 66 samples the amplified processing signals.

(Analyzing Device)

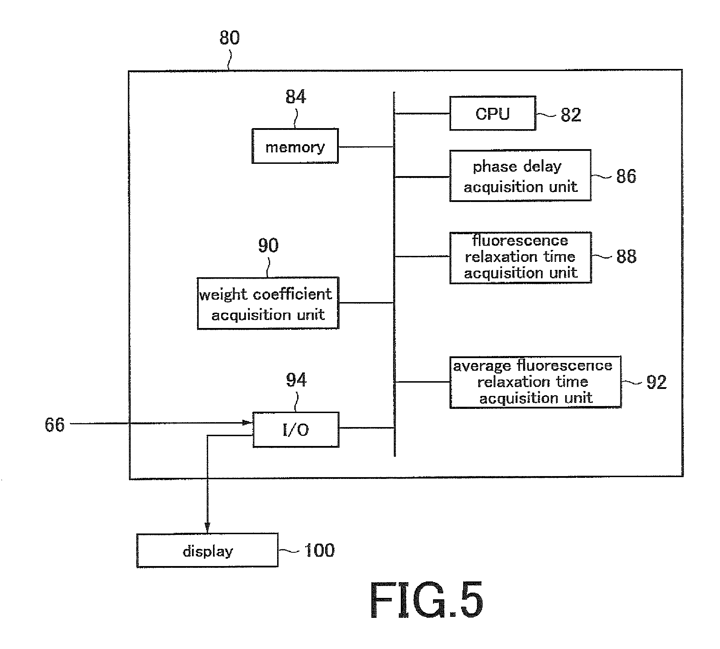

The general structure of the analyzing device (computer) 80 will be described with reference to Fig. 5. Fig. 5 is a schematic diagram illustrating the structure of one example of the analyzing device (computer) 80. The analyzing device 80 is provided by executing a predetermined program on the computer. The analyzing device 80 includes a CPU 82, a memory 84, and an input/output port 94, and further includes a phase delay acquisition unit 86, a fluorescence relaxation time acquisition unit 88, a weight coefficient acquisition unit 90, and an average fluorescence relaxation time acquisition unit 92. These units 86, 88, 90, and 92 are obtained by executing software. The analyzing unit 80 is connected to a display 100.

The CPU 82 is an arithmetic processor provided in the computer. The CPU 82 virtually performs various calculations of the phase delay acquisition unit 86, the fluorescence relaxation time acquisition unit 88, the weight coefficient acquisition unit 90, and the average fluorescence relaxation time acquisition unit 92.

The memory 84 includes a hard disk or ROM that stores a program executed on the computer to provide the phase delay acquisition unit 86, the fluorescence relaxation time acquisition unit 88, the weight coefficient acquisition unit 90, and the average fluorescence relaxation time acquisition unit 92 and a RAM that stores processed results calculated by these units and data supplied from the input/output port 94.

The input/output port 94 receives the input of detected values of the cos components corresponding to at least two frequency components $f_1$ and $f_2$ and of the sin components corresponding to at least two frequency components $f_1$ and $f_2$, which are supplied from the controller 44. The input/output port 94 outputs information about processed results obtained by the units to the display 100. The display 100 displays the values of the processed results obtained by the units such as information about the phase delay of fluorescence, a fluorescence relaxation time, a weight coefficient, and an average fluorescence relaxation time.

The phase delay acquisition unit 86 determines, from the detected values of the cos components corresponding to at least two frequency components $f_1$ and $f_2$ and of the sin components corresponding to at least two frequency components $f_1$ and $f_2$ which are supplied from the controller 44, a phase delay $\theta_{\omega 1}$ at the frequency component $f_1$ (angular frequency $\omega_1$) and a phase delay $\theta_{\omega 2}$ at the frequency component $f_2$ (angular frequency $\omega_2$).

The fluorescence relaxation time acquisition unit 88 determines a fluorescence relaxation time $\tau(\theta_{\omega 1})$ and a fluorescence relaxation time $\tau(\theta_{\omega 2})$ based on the phase delay $\theta_{\omega 1}$ and the phase delay $\theta_{\omega 2}$ determined by the phase delay acquisition unit 86, respectively.

The weight coefficient acquisition unit 90 determines weight coefficients $m(\theta_{\omega 1})$ and $m(\theta_{\omega 2})$ used to assign weights to the fluorescence relaxation times $\tau(\theta_{\omega 1})$ and $\tau(\theta_{\omega 2})$ determined by the fluorescence relaxation time acquisition unit 88, respectively. The weight coefficient is a value of 0 or more but 1 or less.

The average fluorescence relaxation time acquisition unit 92 determines an average fluorescence relaxation time $\tau_{ave}$ based on the fluorescence relaxation times $\tau(\theta_{\omega 1})$ and $\tau(\theta_{\omega 2})$ determined by the fluorescence relaxation time acquisition unit 88 and the weight coefficients $m(\theta_{\omega 1})$ and $m(\theta_{\omega 2})$ determined by the weight coefficient acquisition unit 90.

As described above, by using the detected values of the fluorescent signal corresponding to at least two frequency components $f_1$ and $f_2$, it is possible to determine a fluorescence relaxation time (i.e., the above-described average fluorescence relaxation time $\tau_{ave}$) with high accuracy. The type of the sample 12 is identified by identifying the fluorochrome using the average fluorescence relaxation time $\tau_{ave}$ The reason why higher accuracy of fluorescence relaxation time measurement is achieved by the present invention will be described below in more detail.

The phase delay $\theta$ of the fluorescent signal with respect to the modulation signal for modulating the intensity of laser light generally depends on the fluorescence relaxation time of fluorescence emitted by the fluorochrome. When the phase delay $\theta$ is represented by, for example, a first-order relaxation process, the cos component and the sin component are expressed by the following Equations (1) and (2).

Equation 1

$$\cos \theta = \frac{1}{\sqrt{1 + (\omega \tau)^2}} \quad \cdots (1)$$

Equation 2

$$\sin \theta = \frac{\omega \tau}{\sqrt{1 + (\omega \tau)^2}} \quad \cdots (2)$$

wherein is the modulation angular frequency of laser light and $\tau$ is the fluorescence relaxation time. When the initial fluorescence intensity is defined as $I_0$, the fluorescence relaxation time $\tau$ is a period of time between the time point when the fluorescence intensity is $I_0$ and the time point when the fluorescence intensity becomes $I_0/e$ (e is a base of natural logarithm, e ≈ 2.71828).
The phase delay $\theta$ is determined from the ratio between the cos component and the sin component of the fluorescent signal, that is, tan($\theta$), and the fluorescence relaxation time $\tau$ can be determined by the above Equations (1) and (2) using the phase delay $\theta$.
The tan($\theta$) is represented by the following Equation (3) using the above Equations (1) and (2).
Equation 3

$$\tan \theta = \omega \tau \quad \cdots (3)$$

The phase delay $\theta$ is represented by the following Equation (4) using the above Equation (3).
Equation 4

$$\theta = \tan^{-1}(\omega \tau) \quad \cdots (4)$$

The amount of change ($\delta\theta/\delta\tau$) of the phase delay $\theta$ with respect to the fluorescence relaxation time $\tau$ is represented by the following Equation (5) using the above Equation (4).
Equation 5

$$\frac{\delta \theta}{\delta \tau} = \frac{\omega}{1 + (\omega \tau)^2} \quad \cdots (5)$$

Essentially, if the phase delay $\theta$ can be accurately determined, the fluorescence relaxation time can also be accurately determined, but if the phase delay has an error, the fluorescence relaxation time cannot be determined accurately. However, if the variance of the fluorescence relaxation time with respect to the error of the phase delay is small, the fluorescence relaxation time can be accurately determined with stability. The flow cytometer 10 can accurately determine the fluorescence relaxation time (average fluorescence relaxation time $\tau_{ave}$) by efficiently using a modulation frequency allowing the variance of the fluorescence relaxation time with respect to the variance of the phase delay to be smaller, that is, a modulation frequency allowing the ratio $\delta\theta/\theta\tau$ to be larger.

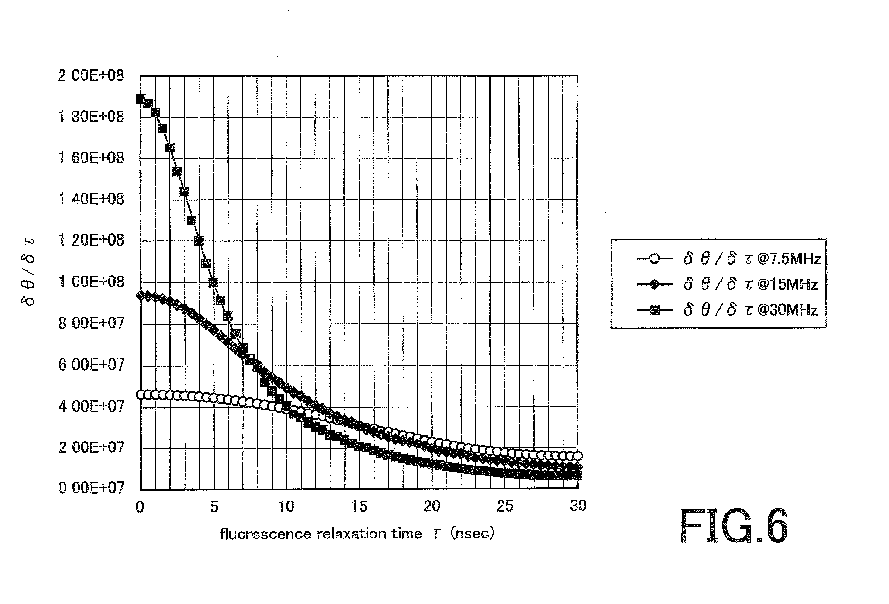

Fig. 6 is a graph illustrating the amount of change ($\delta\theta/\delta\tau$) of the phase delay $\theta$ with respect to the fluorescence relaxation time $\tau$. In Fig. 6, the amounts of change ($\delta\theta/\delta\tau$) of the phase delay $\theta$ when modulation frequencies f are 7.5 MHz, 15 MHz, and 30 MHz are illustrated. The curve obtained when the modulation frequency is 30 MHz and the curve obtained when the modulation frequency is 15 MHz intersect at a point where the fluorescence relaxation time $\tau$ is 7.5 nsec. The curve obtained when the modulation frequency is 15 MHz and the curve obtained when the modulation frequency is 7.5 MHz intersect at a point where the fluorescence relaxation time $\tau$ is 15 nsec.

As can be seen from Fig. 6, when the fluorescence relaxation time $\tau$ is in the range of 0 to 7.5 nsec, the amount of change ($\delta\theta/\delta\tau$) is the largest at a modulation frequency f of 30 MHz, and when the fluorescence relaxation time $\tau$ is in the range of 7.5 to 15 nsec, the amount of change ($\delta\theta/\delta\tau$) is the largest at a modulation frequency f of 15 MHz, and when the fluorescence relaxation time $\tau$ is in the range of 15 or more nsec, the amount of change ($\delta\theta/\delta\tau$) is the largest at a modulation frequency f of 7.5 MHz.

When the amount of change ($\delta\theta/\delta\tau$) of the phase delay $\theta$ is larger, a higher S/N ratio is obtained and therefore higher measurement accuracy is achieved. For this reason, when the fluorescence relaxation time of the measurement object extends over a wide range, modulation of the intensity of laser light only at, for example, 30 MHz decreases an S/N ratio in a range where the fluorescence relaxation time exceeds 20 nsec, which makes it difficult to achieve satisfactory measurement accuracy.

In this embodiment, as described above, weights are assigned to the fluorescence relaxation times at two frequencies to determine the average of the fluorescence relaxation times. That is, the oscillating frequency $f_1$ of the oscillator 46a is set to 30 MHz and the oscillating frequency $f_2$ of the oscillator 46b is set to 15 MHz, and the phase delay $\theta_{\omega 1}$ at the frequency $f_1$ and the phase delay $\theta_{\omega 2}$ at the frequency $f_2$ are determined.

Then, the average fluorescence relaxation time acquisition unit 92 determines the average fluorescence relaxation time $\tau_{ave}$ by the following Equation (6), that is, by multiplying each of the fluorescence relaxation times $\tau(\theta_{\omega 1})$ and $\tau(\theta_{\omega 2})$ by the weight coefficient $m(\theta_{\omega i})$. Here, N is an integer of 2 or more, and represents the number of different frequency components. In this embodiment, N is 2. The weight coefficient acquisition unit 90 determines the weight coefficient $m(\theta_{\omega i})$ that increases as the amount of change ($\delta\theta/\delta\tau$) of the phase delay $\theta$ increases when the fluorescence relaxation time $\tau$ has a certain value.

$$\text{Equation 6}$$

$$\tau_{ave} = \sum_{i=1}^{N} m(\theta_{\omega_i})\tau(\theta_{\omega_i}) \quad \cdots(6)$$

Here, the fluorescence relaxation time $\tau$ (7.5 nsec) at the intersection point of the curve obtained when the modulation frequency is 30 MHz and the curve obtained when the modulation frequency is 15 MHz corresponds to a phase delay $\theta_{\omega 1}$ of 0.9555 [rad] and to a phase delay $\theta_{\omega 2}$ of 0.6153 [rad]. Therefore, the weight coefficient acquisition unit 90 determines the weight coefficients $m(\theta_{\omega 1})$ and $m(\theta_{\omega 2})$ so that $m(\theta_{\omega 1})$ and $m(\theta_{\omega 2})$ satisfy $m(\theta_{\omega 1}) > m(\theta_{\omega 2})$ when the fluorescence relaxation time $\tau$ is shorter than 7.5 nsec ($0 \le$ phase delay $\theta_{\omega 1} < 0.9555$ [rad]). Further, the weight coefficient acquisition unit 90 determines the weight coefficients $m(\theta_{\omega 1})$ and $m(\theta_{\omega 2})$ so that $m(\theta_{\omega 1})$ and $m(\theta_{\omega 2})$ satisfy $m(\theta_{\omega 1}) < m(\theta_{\omega 2})$ when the fluorescence relaxation time $\tau$ is 7.5 nsec or longer (0.6153 [rad] $\le$ phase delay $\theta_{\omega 2} < \pi/2$ [rad]). The value of the phase delay allowing the magnitude relationship between the weight coefficients to be reversed depends on the modulation frequencies $f_1$ and $f_2$ used. Therefore, weight coefficients satisfying the above-mentioned magnitude relationship are previously stored in the memory 84.

The type of the sample 12 can be identified with higher accuracy by identifying the fluorochrome using the average fluorescence relaxation time $\tau_{ave}$.

Second Embodiment

As described above, the flow cytometer according to the first embodiment has a structure in which two oscillators different in oscillating frequency are provided. However, a flow cytometer according to a second embodiment may have a structure in which three or more oscillators different in oscillating frequency are provided. In the second embodiment, three oscillators are provided, and the frequency $f_1$ is set to 30 MHz, the frequency $f_2$ is set to 15 MHz, and the frequency $f_3$ is set to 7.5 MHz. The phase delay acquisition unit 86 determines the phase delay $\theta_{\omega 1}$ at the frequency $f_1$, the phase delay $\theta_{\omega 2}$ at the frequency $f_2$, and the phase delay $\theta_{\omega 3}$ at the frequency $f_3$.

Then, the average fluorescence relaxation time acquisition unit 92 determines the average fluorescence relaxation time $\tau_{ave}$ by the above Equation (6), that is, by multiplying each of the fluorescence relaxation times $\tau(\theta_{\omega 1})$ $\tau(\theta_{\omega 2})$, and $\tau(\theta_{\omega 3})$ by the weight coefficient $m(\theta_{\omega i})$. In this embodiment, N is 3. The weight coefficient acquisition unit 90 determines the weight coefficient $m(\theta_{\omega i})$ that increases as the amount of change $(\delta\theta/\delta\tau)$ of the phase delay $\theta$ increases when the fluorescence relaxation time $\tau$ has a certain value.

Here, the fluorescence relaxation time $\tau$ (7.5 nsec) at the intersection point of the curve obtained when the modulation frequency is 30 MHz and the curve obtained when the modulation frequency is 15 MHz corresponds to a phase delay $\theta_{\omega 1}$ of 0.9555 [rad] and to a phase delay $\theta_{\omega 2}$ of 0.6153 [rad], and the fluorescence relaxation time $\tau$ (15 nsec) at the intersection point of the curve obtained when the modulation frequency is 15 MHz and the curve obtained when the modulation frequency is 7.5 MHz corresponds to a phase delay $\theta_{\omega 2}$ of 0.9555 [rad] and to a phase delay $\theta_{\omega}3$ of 0.6153 [rad].

Therefore, the weight coefficient acquisition unit 90 determines the weight coefficients $m(\theta_{\omega 1})$, $m(\theta_{\omega 2})$, and $m(\theta_{\omega 3})$ so that $m(\theta_{\omega 1})$, and $m(\theta_{\omega 3})$ satisfy $m(\theta_{\omega 1}) > m(\theta_{\omega 2})$, $m(\theta_{\omega 3})$ when the fluorescence relaxation time $\tau$ is shorter than 7.5 nsec ($0 \leq$ phase delay $\theta_{\omega 1} < 0.9555$ [rad]). Further, the weight coefficient acquisition unit 90 determines the weight coefficients $m(\theta_{\omega 1})$, $m(\theta_{\omega 2})$, and $m(\theta_{\omega 3})$ so that $m(\theta_{\omega 1})$, $m(\theta_{\omega 2})$, and $m(\theta_{\omega 3})$, satisfy $m(\theta_{\omega 2}) > m(\theta_{\omega 1})$, $m(\theta_{\omega 3})$ when the fluorescence relaxation time $\tau$ is 7.5 nsec or longer but shorter than 15 nsec (0.6153 [rad] $\leq$ phase delay $\theta_{\omega 2} < 0.9555$ [rad]). Further, the weight coefficient acquisition unit 90 determines the weight coefficients $m(\theta_{\omega 1})$, $m(\theta_{\omega 2})$, and $m(\theta_{\omega 3})$ so that $m(\theta_{\omega 1})$, $m(\theta_{\omega 2})$, and $m(\theta_{\omega 3})$ satisfy $m(\theta_{\omega 3}) > m(\theta_{\omega 1})$, $m(\theta_{\omega 2})$ when the fluorescence relaxation time $\tau$ is 15 nsec or longer (0.6153 [rad] $\leq$ phase delay $\theta_{\omega 3} < \pi/2$ [rad]).

The type of the sample 12 can be identified with higher accuracy by identifying the fluorochrome using the average fluorescence relaxation time $\tau_{ave}$.

Third Embodiment

As can be seen from the above Equation (5), in a region where the fluorescence relaxation time $\tau$ is small, the amount of change $(\delta\theta/\delta\tau)$ of the phase delay $\theta$ increases as the modulation angular frequency $\omega$ increases, but on the other hand in a region where the fluorescence relaxation time $\tau$ is large, the amount of change $(\delta\theta/\delta\tau)$ of the phase delay $\theta$ increases as the modulation angular frequency $\omega$ decreases.

Therefore, it is preferred that the different frequencies of two or more oscillators used are not relatively close to each other, that is, the difference between the different frequencies of two or more oscillators used is large. For example, the value of one of at least two frequencies is preferably two times or larger than that of the other frequency.

By setting one of at least two frequencies to a value two times or larger than that of the other frequency, it is possible to identify the type of the sample 12 with higher accuracy.

Fourth Embodiment

As described above, in the second embodiment, the magnitude relationship among the weight coefficients $m(\theta_{\omega 1})$, $m(\theta_{\omega 2})$, and $m(\theta_{\omega 3})$ is defined according to the fluorescence relaxation time $\tau$. A fourth embodiment is different from the second embodiment in that the weight coefficients $m(\theta_{\omega 1})$, $m(\theta_{\omega 2})$, and $m(\theta_{\omega 3})$ are set to either 0 or 1, but the other structures are the same as those in the second embodiment.

More specifically, the weight coefficient acquisition unit 90 determines the weight coefficients $m(\theta_{\omega 1})$, $m(\theta_{\omega 2})$, and $m(\theta_{\omega 3})$ so that $m(\theta_{\omega 1}) = 1$ and $m(\theta_{\omega 2}) = m(\theta_{\omega 3}) = 0$ when the fluorescence relaxation time $\tau$ is shorter than 7.5 nsec ($0 \leq$ phase delay $\theta_{\omega 1} < 0.9555$ [rad]). Further, the weight coefficient acquisition unit 90 determines the weight coefficients $m(\theta_{\omega 1})$, $m(\theta_{\omega 2})$, and $m(\theta_{\omega 3})$ so that $m(\theta_{\omega 2}) = 1$ and $m(\theta_{\omega 1}) = m(\theta_{\omega 3}) = 0$ when the fluorescence relaxation time $\tau$ is 7.5 nsec or longer but shorter than 15 nsec (0.6153 [rad] $\leq$ phase delay $\theta_{\omega 2} < 0.9555$ [rad]). Further, the weight coefficient acquisition unit 90 determines the weight coefficients $m(\theta_{\omega 1})$, $m(\theta_{\omega 2})$, and $m(\theta_{\omega 3})$ so that $m(\theta_{\omega 3}) = 1$ and $m(\theta_{\omega 1}) = m(\theta_{\omega 2}) = 0$ when the fluorescence relaxation time $\tau$ is 15 nsec or longer (0.6153 [rad] $\leq$ phase delay $\theta_{\omega 3} < \pi/2$ [rad]).

The kind of the sample 12 can be identified with higher accuracy by identifying the fluorochrome using the average fluorescence relaxation time $\tau_{ave}$.

Description of Reference Numerals

10                              flow cytometer

| 12 | sample |
| 20 | signal processing unit |
| 22 | laser light source unit |
| 23 | light source |
| 24a, 24b | lens systems |
| 25, 26 | light-receiving units |
| 27 | photoelectric converter |
| 28 | control/processing unit |
| 30 | tube |
| 32 | collection vessel |
| 34 | laser driver |
| 40 | signal generation unit |
| 42 | signal processing unit |
| 44 | controller |
| 46a, 46b | oscillators |
| 48a, 48b, 48d | power splitters |
| 48c | power divider |
| 50a, 50b, 50c, 54, 55, 64 | amplifiers |
| 58, 59 | IQ mixers |
| 60 | system controller |
| 62 | low-pass filter |
| 66 | A/D converter |
| 80 | analyzing device |
| 82 | CPU |
| 84 | memory |
| 86 | phase delay acquisition unit |
| 88 | fluorescence relaxation time acquisition unit |
| 90 | weight coefficient acquisition unit |
| 92 | average fluorescence relaxation time acquisition unit |
| 94 | input/output port |
| 100 | display |

**Claims**

1. A fluorescence detecting device for receiving fluorescence emitted by a measurement object by irradiating the measurement object with laser light and processing a fluorescent signal obtained at this time, the device comprising:

   a laser light source unit configured to irradiate the measurement object with laser light;
   a light-receiving unit configured to output a fluorescent signal of fluorescence emitted by the measurement object irradiated with the laser light;
   a light source control unit configured to generate a modulation signal for time-modulating an intensity of the laser light emitted from the laser light source unit by at least two frequency components; and
   a processing unit configured to determine a fluorescence relaxation time of the fluorescence emitted by the measurement object by using the fluorescent signal outputted by the light-receiving unit and the modulation signal,
   wherein the processing unit is configured to determine phase delays of the fluorescent signal with respect to the modulation signal at the two frequency components, to determine a fluorescence relaxation time at each of the frequency components by using the phase delay, and to determine an average fluorescence relaxation time by weighted averaging of the fluorescence relaxation times.

2. The fluorescence detecting device according to claim 1, wherein the modulation signal is a signal obtained by combining signals of at least two frequencies.

3. The fluorescence detecting device according to claim 1 or 2, wherein the processing unit is configured to determine the average fluorescence relaxation time by using a weight coefficient determined according to an amount of change of the phase delay at each of the at least two frequency components with respect to the fluorescence relaxation time.

4. The fluorescence detecting device according to any one of claims 1 to 3,
wherein one of the at least two frequencies has a value two times or larger than that of another frequency.

5. A fluorescence detecting method by receiving fluorescence emitted by a measurement object by irradiating the measurement object with laser light and processing a fluorescent signal obtained at this time, the method comprising the steps of:

irradiating the measurement object with laser light;
outputting a fluorescent signal of fluorescence emitted by the measurement object irradiated with the laser light;
generating a modulation signal for time-modulating an intensity of the laser light by at least two frequency components; and
determining a fluorescence relaxation time of the fluorescence emitted by the measurement obj ect by using the fluorescent signal and the modulation signal,
wherein the step of determining a fluorescence relaxation time includes the step of determining phase delays of the fluorescent signal with respect to the modulation signal at the two frequency components, the step of determining a fluorescence relaxation times at each of the frequency components by using the phase delay, and the step of determining an average fluorescence relaxation time by weighted averaging of the fluorescence relaxation times.

6. The fluorescence detecting method according to claim 5, wherein in the step of generating a modulation signal, signals of the at least two frequency components are combined.

7. The fluorescence detecting method according to claim 5 or 6, wherein in the step of determining a fluorescence relaxation time, the average fluorescence relaxation time is determined by using a weight coefficient determined according to an amount of change of the phase delay at each of the at least two frequency components with respect to the fluorescence relaxation time.

8. The fluorescence detecting method according to any one of claims 5 to 7, wherein one of the at least two frequencies has a value two times or larger than that of another frequency.

sheath liquid

sample

12

30

26

25

22

20

28

control/
processing unit

32

analyzing device
(computer)

10

80

# FIG.1

FIG.2

EP 2 397 841 A1

EP 2 397 841 A1

**FIG.3**

FIG.4

40:signal generation unit

50c     48c    48a    46a

28

34 ← AMP ← power divider ← power splitter ← f1 oscillator

50a      46b

AMP ← power splitter ← f2 oscillator

50b

48b

48d    54     58

27 → power splitter → AMP → IQ mixer ← f1

55     59

AMP → IQ mixer ← f2

42:signal processing unit

trigger signal   25:light-receiving unit

low-pass filter ~62     system controller ~60

AMP → A/D → 80:analyzing device (computer)

44:controller    64     66

EP 2 397 841 A1

15

FIG.5

EP 2 397 841 A1

FIG.6

## INTERNATIONAL SEARCH REPORT

| International application No. |
| --- |
| PCT/JP2010/000390 |

**A. CLASSIFICATION OF SUBJECT MATTER**
*G01N21/64*(2006.01)i

According to International Patent Classification (IPC) or to both national classification and IPC

**B. FIELDS SEARCHED**

Minimum documentation searched (classification system followed by classification symbols)
G01N21/62-G01N21/83, G01N33/48-G01N33/98, G01N15/00-G01N15/14

Documentation searched other than minimum documentation to the extent that such documents are included in the fields searched
    Jitsuyo Shinan Koho          1922-1996   Jitsuyo Shinan Toroku Koho   1996-2010
    Kokai Jitsuyo Shinan Koho    1971-2010   Toroku Jitsuyo Shinan Koho   1994-2010

Electronic data base consulted during the international search (name of data base and, where practicable, search terms used)
    JSTPlus(JDreamII), JMEDPlus(JDreamII), JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2007-501414 A  (Dakota Technologies, Inc.), 25 January 2007 (25.01.2007), paragraph [0056] & US 2004/0007675 A1    & EP 1620713 A & WO 2004/102155 A2    & CA 2524521 A | 1-8 |
| A | JP 2000-304697 A  (Laboratory of Molecular Biophotonics), 02 November 2000 (02.11.2000), paragraph [0059] (Family: none) | 1-8 |
| A | JP 2005-114528 A  (Olympus Corp.), 28 April 2005 (28.04.2005), paragraph [0032] & US 2005/0072936 A1    & EP 1522833 A1 | 1-8 |

☐ Further documents are listed in the continuation of Box C.      ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search 04 February, 2010 (04.02.10) | Date of mailing of the international search report 16 February, 2010 (16.02.10) |
| --- | --- |
| Name and mailing address of the ISA/ Japanese Patent Office | Authorized officer |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006226698 A